# EUROPEAN PATENT APPLICATION

(11) **EP 2 857 011 A1**
(43) Date of publication of application: **08.04.2015**
(21) Application number: 12877152.4
(22) Date of filing: 31.10.2012
(51) Int. Cl.: A61K 31/13, A61K 31/4045, A61K 9/20, A61K 9/48, A61P 25/00

(54) **PHARMACEUTICAL COMPOSITION FOR THE PROPHYLAXIS AND TREATMENT OF PSYCHOLOGICAL, BEHAVIORAL AND COGNITIVE DISORDERS**

(30) Priority: 24.05.2012 RU 2012121410
(71) Applicant: Ltd. "Valenta-Intellekt", Moscow 119530 (RU)
(72) Inventor: MOROZOVA, Margarita Alekseevna, 105187 Moskva (RU); BENIASHVILI, Allan Gerovich, 109044 Moskva (RU); ZAPOLSKY, Maxim Eduardovich, Lyuberetskiy raion , pos. Tomilino 140073 Moskovskya oblast (RU)
(74) Representative: Glawe, Delfs, Moll
(86) International application number: PCT/RU2012/000888
(87) International publication number: WO 2013/176567

(57) **Abstract**

The invention relates to the field of medicine and the pharmaceutical-chemical industry, and specifically to the field of neurology, and concerns new compositions containing memantine and melatonin. It has been established that a strengthening of the effect of memantine takes place when it is combined with melatonin. The invention may be realized through the preparation of a finished pharmaceutical product such as capsules, preferably hard gelatin capsules.

## Description

### Field of the Invention

The present invention relates to the fields of medicine and chemical and pharmaceutical industry, in particular to the field of neurology, and is aimed at new compositions comprising memantine and melatonin.

### Prior Art

Memantine is a medium-affinity voltage-dependent noncompetitive NMDA receptor antagonist. Memantine blocks effects of a pathologically raised glutamate level that may cause neuron dysfunction. It provides neuroprotective, antispasmogenic, antiparkinsonian actions. It inhibits glutamatergic neurotransmission and progress of neurodegenerative processes, provides neuromodulating action. It promotes normalization of mental activity, improves memory, raises ability to concentrate attention and correct motor disorders [Ditzler K. Efficacy and tolerability of memantine in patients with dementia syndrome. A double-blind, placebocontrolled trial. Arzneimittel-Forschung. 1991; 41: 773-80.]. Apart from the main mechanism of action (influence on glutamatergic neurotransmission), memantine has an additional serotonergic effect (noncompetitive antagonist of 5HT3-receptors) and, possibly, its antidepressant effect is conditioned by just this mechanism. The antidepressant action of memantine has been clinically shown in a number of studies on a high-depression model [Ferguson JM, Shingleton RN. An open-label, flexible-dose study of memantine in major depressive disorder. ClinNeuropharmacol 2007; 30 (3): 136-44.].

After a single dosing of memantine, its concentration peak in the brain is achieved in one hour. The half-life of memantine is rather long, being approximately 100 hours. This long half-life allows to suppose that this pharmaceutical substance has the accumulation effect when administered chronically. Thus, after a single dosing of memantine the dopamine concentration remains unchanged, but during prolonged administration a dopamine concentration in the forebrain regions raises 10 times or more. The longer is memantine administration, the higher is dopamine concentration. An analysis of dopamine metabolite concentrations shows that the use of memantine results not only in the release, but also in the synthesis of dopamine [Hesselink MB, DeBoer AG, Breimer DD et al. J Neural Transm 1999; 106: 803-18.].

It is known from literature that chronic administration of memantine has an effect on concentration increase of not only dopamine, but also acetylcholine, as well as promotes an increase in a number of muscarine cerebral receptors. Thus, it may be supposed that this preparation provides a complex mediator action both on the nerve cells of frontal lobe cortex and on the entorhinal cortex and its associations with the parietotemporal regions of the brain.

The second aspect of the memantine therapeutic activity is its neuroprotective action. This effect is a direct result of blocking the NMDA-receptors, closing the ionotropic channels and, correspondingly, stabilizing the cell membranes, which protects cells against their death. The memantine neuroprotective action has been proved on a cerebral ischemia model in an experiment [Danysz W, Parsons CG, Möbius H-J et al. NeurotoxRes 2000; 2: 85-97.]. The preventive administration of memantine reduces the ischemic penumbra area, manifestation of a cerebral edema and reduces a surface area of an ischemia focus itself. A similar result has been obtained on other models of cerebral affection. It is supposed that memantine may produce a stimulating effect on synthesis of a number of neurotrophic factors, in particular of the brain-derived neurotrophic factor, which leads to stabilization of the neuronal membrane and protection of a cell against death. It is shown that the use of NMDA receptor antagonists has a positive effect on the patient cognitive functions at the Parkinson disease [Damulin I.V. Rus. Med. Journ. 2001; 9 (25): 1178-82.; Litvinenko I.V., Odinak M.M.. Journ. of Neurol. and Psych. named after S.S. Korsakov. 2004;4:76-81.].

Memantine (1-amino-3,5-dimethyladamantan) is an analogue to 1-amino-cyclohexane. Its formula is disclosed, for example, in US Patents Nos. 4,122,193; 4,273,774; 5,061,703. Memantine and other 1-amino-cyclohexanes have proved their usability for ameliorating various progressive neurodegenerative disorders, such as dementia in patients suffering from moderate and severe Alzheimer's disease, Parkinson's disease and muscle spasticity, as disclosed in US Patents Nos. 5,061,703; 5,614,560 and 6,034,134.

Melatonin (N-acetyl-5-methoxytriptamine) is a neuropeptide that is synthesized mainly by the epiphysis and has a number of unique properties for the organism of the humans and mammals. Of significance is the fact of circadian period of producing biologically active compounds in the pinealocyte. The melatonin synthesis is effective only with the beginning of the dark time and drops in the light phase of the day - this fact was first shown by R. Wurtman in 1960. For suppressing this process a short light pulse (0.1-1 lux) is sufficient. During the daytime, on the contrary, serotonin is accumulated in the tissue of this gland. The daily rhythm of the melatonin production depends on the NAT activity in the retina, which, in its turn, depends on ions of calcium, dopamine and gamma-aminobutyric acid (GABA). The epiphysis uses melatonin for organizing daily rhythm and for regulating cyclic processes, acting as the mediator between the pace-making mechanism of the suprachiasmatic nuclei (SCN) and peripheral organs. The epiphysis, together with the SCN of the hypothalamus, is a part of the system of the so-called organism biological clock playing a key role in the mechanism of "counting the inner time" and ageing [Arushanyan E.B., 2005; Anisimov V.N., 2007]. The main functions of the epiphysis in the organism are: regulation of circadian and seasonal rhythms; regulation of the reproductive function; antioxidant protection and anticancer protection [Anisimov V.N., 1998, 2003]. Though the main source of melatonin circulating in the blood is the epiphysis, the paracrine synthesis of melatonin is found practically in all the organs and tissues, such as the thymus, the gastrointestinal tract, the gonads, the connective tissue [Reiter R.J.; Reikhlin I.M., Kvetnoy I.M.; Huether G.].

Melatonin (N-acetyl-5-methoxytriptamine) is an indole compound produced by the epiphysis, the retina and the intestine.

The epiphysis of the humans and animals, also as the thymus, involutes with age. A number of active secretory elements (pinealocytes) of the gland is reduced, and the melatonin production is lowered. Approximately by 45 years of age the plasma contains only a half of the amount of this hormone that is produced in youth. Furthermore, the amplitude and dynamics oft he daily melatonin secretion are changed in elderly persons [Korkushko O.V., Khavinson V.Kh., Shatilo V.B. The pineal gland: ways of correction during ageing. SPb. Nauka Publishers, 2006. 204 pp.; Reiter R.J. The aging pineal gland and its physiological consequences //BioEssays, 1992. V. 14. p. 169-175., Skene D.J., Swaab D.F. Melatonin rhythmicity: effect of age and Alzheimer disease //Exp. Gerontol. 2003. V.38. p.199-206]. During experiments on animals and observations of therapies of elderly persons, both in the normal condition, and in various forms of cerebral pathologies, including those of vascular nature, melatonin improves the processes of memory, visual and aural perception, concentration of attention. These facts determine treatment possibilities of melatonin as a potential nootropic agent [Arushanyan E.B.. The epiphysial hormone melatonin - a novel nootropic agent? //Exper. and Clin. Pharmacol. 2005; V.68. p.74-79.; Arushanyan E.B.. The epiphysial hormone melatonin and neurological pathology //Rus. Med. Journ. 2006. V. 14. p.1057-1063].

Melatonin invariably produced a distinct neuroprotective effect in experiments on models of acute disturbance of brain hemodynamics in animals: total or focal ischemia due to artery occlusions, photothrombosis [Reiter R.J., Tan D.X, Leon J. et al. When melatonin gets of your nerves: its beneficial actions in experimental models of stroke //Exp. Biol. Med. 2005. V.230. p. 104-117.]. The application of the hormone to mice on a model of a closed head injury accelerated normalization of the animal behavior and restorative processes in the injury focus in the form of reducing its dimensions together with increasing plasma content of a number of antioxidants including ascorbic acid [Beni S.M., Kohen R., Reiter R.J. et al. Melatonin-induced neuroprotection after closed head injury is associated with increased brain antioxidants and attenuated late-phase activation of NF-kB and AP-1 //FASEB J. - 2004. - V.18. - p. 149-151., Mesenge C, Verrecchia C, Boulu R.G. Neuroprotection by melatonin in mice submitted to brain injury //Naunyn. Schmiedeberg'sArch.Pharmacol. - 1998. V.358. - p.R32/].

Data obtained with the use of visualization objective methods (nuclear magnetic resonance) show that the second administration of melatonin to rats reduces the volume of cereblar edema 2 times [Torii K., Uneyama H., Nishino H. Melatonin suppresses cerebral edema caused by middle cerebral artery occlusion/reperfusion in rats assessed by magnetic resonance imaging.// J.PinealRes. - 2004. - V.36. - p. 18-24.]. Accounting of the neuron morphometric characteristics of various cerebral formations, such as neocortex, hippocampus, striatum, with data of NMR-tomography enables to reveal a statistically-significant protective effect of the epiphyseal hormone, where its use enabled to keep a significant number of cell elements vital, the manifestation of a cerebral edema being significantly limited.

The fact is shown that an edema in the cerebral cortex failed quicker than in the striate body [Kondoh T., Uneyama N., Nishino H. Melatonin reduces cerebral edema formation caused by transient for ebrainis chemiak in rats //LifeSci. -.2002. - V.72. - p.583-590., Regriny O., Delagrange P., Scalbert E. et al. Melatonin improves cerebral circulation security margin in rats //Am. J. Physiol. - 1998. - V.275. - p.H139-H134., Sinha K., Degaonkar M.N., Jagannathan N.R. Effect of melatonin on ischemia/reperfusion injury induced by middle cerebral artery occlusion in rats //Eur.J. Pharmacol. - 2002. - V.428. - p.185-192.]. There is a supposition that melatonin is evolutionarily included into the natural system of the brain protection against ischemic injury, which is evidenced by results of experiments on animals in which a hormone deficit has been produced by various methods, such as extirpation of the epiphysis, keeping at bright illumination for a long time. Focuses of cerebral tissue infarctions are much more manifested in these conditions, when a stroke is modeled by cortical photothrombosis or a local occlusion of the median cerebral artery. And at the same time, melatonin injections at the background of the same epiphysis ectomy resulted in reliable protection of cortical neurons against ischemia of various etiologies [Kilic E., Ozdemir Y., Bolay H. Pinealectomy aggravates and melatonin administration attenuates brain damage in focal ischemia. II]. Cerebr. BloodFlowMetabol. - 1999. - V.19. - p.511-516.].

A pathological hyperactivity of glutamic acid is recognized as a pronounced factor of an oxidant stress in disorders of the cerebral blood circulation due to atherosclerosis, stroke or a craniocerebral injury [Gusev Ye.I., Skvortsova V.I.. Glutamate neurotransmission and calcium metabolism in the normal state and at cerebral ischemia //Successes of Physiol. Sciences. - 2002. - V.33. - p.80-93.]. Accumulation of glutamate in synapses and the intercellular space leads to launching a glutamate-calcium cascade.

By stimulating N-methyl-D-aspartate (NMDA) receptors glutamate opens channels in neuronal membranes for calcium ions, causing their intracellular accumulation in great quantities, which inevitably leads to damages of cellular structures. Melatonin distinctly inhibits glutamate neurotoxicity. As determined for a culture of isolated cortical neurons, their damage at excess of glutamate or NMDA is significantly inhibited after adding melatonin into an incubation medium. This is, to a certain extent, due to its ability of binding calmodulin and restrict the function of NMDA-receptors. The epiphysis ectomy leads to an increased density of NMDA receptors together with simultaneous strengthening of lipid peroxidation in various cerebral formations. Melatonin protects neurons against aggression of NO which excess may make glutamate neurotoxicity more potent [GuerreroJ.M., ReiterR.J., OrtizG. etal. Melatonin prevents increases in neuronal nitric oxide and cyclic GMP production after transient brain ischemia and reperfusion in the Mangolian gerbil //J.Pineal Res. - 1997. - V.23. - p.24-31.].

Also, melatonin normalizes the mitochondrion activity [El-Abhar H.S., Shaalan M., Barakat M. et al. Effect of melatonin and nifedipine on some antioxidant enzymes and different energy fuels in the blood and brain of global ischemic rats//J.Pineal Res. -2002. - V.33. - p.87-94.] and not only limits defects of the mitochondrial function, protein hyperphosphorylation and cytoskeleton disorganization due to hypoxia, but also restores the function of the tyrosine kinase receptor apparatus that is an important element of the phosphorylation system and participates in reparative processes in the nerve tissue by involving neurotrophins thereinto [Olivieri G., Otten U., Meier F. et al. Beta-amyloid modulates tyrosine kinase B receptor expression in SH SY SY neuroblastome cells: influence of the antioxidant melatonin //Neurosci. - 2003. - V.120. - p. 659-665.]. Melatonin, possibly, stimulates neurogenesis in the mature nerve tissue also. It has been shown for a culture of stem cells that they may express melatonin receptors, primarily of the first type. Addition of a low-concentration melatonin solution to them provokes, *inter alia,* the induction of mRNA of one of the neurotrophins, namely the glial cell-line-derived neurotrophic factor (GDNF) [Niles L.P., Armstrong K.J., RinconCastro L.M. et al. Neural stem cells express melatonin receptors and neurotrophic factors: colocalization of the MT 1 receptors with neuronal and glial markers //BMC Neurosci. - 2004. - V.5. - p. 41-50.].

Patent documents are known that disclose the use of melatonin, such as RU Patent No. 2268737 "Method for treating atopic dermatitis", by administering melatonin in the unit dose of 3 mg, at 9 P.M., the course is 21 days; RU Patent No. 2428183 "Melatonin applications as an adaptogen"; RU Patent No. 2418586 "Method for correcting disorders in the reproductive organs by administering melatonin"; RU Patent No. 2394571 "Method for treating such inflammatory diseases by administering melatonin 40 minutes before sleep"; RU Patent No. 2336890 "Compounds comprising melatonin, ginkgo biloba and biotin" discloses a compound for stimulating hair growth; RU Patent No. 2294741 "Method for treating patients with ischemic heart disease" discloses the use of melatonin at the background of standard therapy"; RU Application No. 2008150624 (PCT US Application 373.06.2007 20070521) "Treatment of depressive disorders"; RU Application No. 2009141713 (PCT US Application 100.06.2008 20080411) "Compositions protecting against ischemia/reperfusion" where melatonin is mentioned in a combination; RU Application No. 2009137472 "Melatonin tablet and methods for producing and using same" that discloses melatonin in a dissolved state in association with a pharmaceutically acceptable carrier; RU Application No. 97113435 "Method for treating drug addiction".

A preparation comprising memantine is known that is used for treating dementia and has the form of tablets enclosed in a film coating. These tablets comprise memantine hydrochloride - 10 mg, adjuvants: colloid silicon dioxide (Aerosil) 3 mg; calcium hydrophosphate dehydrate 50.4 mg; sodium cross-carmellose (Primellose) 3 mg; lactose monohydrate (milk sugar) 136 mg; magnesium stearate 1.6 mg; povidone 6 mg; coating composition: SeleCoat AQ-02003 6 mg, including: hypromellose (hydroxymethylpropylcellulose) 3.6 mg; Macrogol-6000 (polyethylene glycol 6000) 1.2 mg; titanium oxide 1.2 mg. (http://www.canonpharma.ru/ru/drugs/expert/doctors/neurology/m emantinkanon/).

VITA-MELATONIN® preparation is known that is produced by the Kyiv Vitamin Factory, the preparation comprising: Melatonin 3 mg, other ingredients: milk sugar, microcrystalline cellulose, potato starch, calcium stearate. The preparation is intended for prophylaxis and treatment of "sleep-wakefulness" circadian rhythm disorders occurring when time zone changes and manifesting as increased fatigability; sleep disorders including chronic insomnia of functional origin, insomnia in elderly persons (including that accompanied by arterial hypertension and hypercholesterolemia); for improving mental and physical ability to work as well as for eliminating stress reactions and depressions having seasonal nature. Hypertension and idiopathic hypertension (of I-II stages) in elderly patients (included into complex therapy) (http://compendium.com.ua/info/67093).

RU Patent No. 2326660 C1 may be indicated as the closest analogous solution, the said patent relates to production of oral preparation dosage forms having neuroprotective action, in particular the Memantin preparation being a modulator of the glutomatergic system and being used for treating dementias, memory impairment, cerebral and spinal spastic syndrome. The Memantin preparation is in the form of capsules, a therapeutically efficient quantity of memantine being included into the composition of a mass filling said capsules, and the said mass being a mixture of powders or a granulated material. The mass in capsules contains, in addition, adjuvants, a physiologically acceptable excipient as well as, when necessary, a disintegrating agent and an anti-friction substance.

### Summary of the Invention

The objective of the invention is to create solid dosage forms comprising memantine and melatonin in a fixed combination possessing high therapeutic properties and reduced side effects.

This objective is achieved by new preparation dosage forms, wherein the content of active substances may reach a very high value, nearly 90% or even more, with the use of minimum adjuvants that are affordable as to their cost and that ensure economically beneficial production on the industrial scale. The present invention gives the possibility of obtaining the said important technical effects.

Thus, keeping the number of adjuvants at a minimum, elimination, but not exclusively, of the use of organic solvents, decreasing a number of stages and reducing production time and cost, form pre-requisites to the invention.

The invention object is a pharmaceutical composition intended for prophylaxis and treatment of mental, behavioral, and cognitive disorders and having a solid dosage form comprising memantine and melatonin as the active basis and adjuvants including at least one diluent selected from lactose, starch, starch derivative, microcrystalline cellulose, sucrose, inverted sugar, dextrose and dextrate; at least one disintegrating agent selected from sodium carboxymethylcellulose, cross-carmellose, jellied starch; a binding agent selected from polyvynilpirrolidon, gelatin, cellulose derivatives, natural gums, polyethylene glicols, sodium alginate; an anti-friction agent selected from stearic acid and/or its salts, colloid silicon dioxide, talk, sodium benzoate, sodium acetate and sodium oleate, at the following component ratio, in weight %:
memantine 40.0 - 90.0
melatonin 2.0 - 5.0
diluent 2.0 - 50.0
binding agent 3.5 - 10.0
disintegrating agent 1.5 - 10.0
anti-friction agent 0.2 - 3.0

In order to produce the preparation, in particular a memantine and melatonin composition, memantine and melatonin are mixed with various adjuvants for producing a solid form. In some embodiments the solid form is a tableted form, in other embodiments it is a capsule or powder intended for producing a solution for oral administration.

An additional aspect of this invention comprises a method for producing preparation forms of the claimed composition. This method provides for producing a solid dosage form of the claimed composition, preferably by wet mixing of the active ingredients and adjuvants with water, then the granulated mixture is dried and ground. The claimed invention also includes the use of these compositions for treating a disease and/or disorder in persons in need of this, which treatment includes oral administration of a therapeutically efficient amount of a composition according to this invention.

In order to work this invention, but not exclusively, a composition is proposed that comprises memantine and melatonin, but does not comprise magnesium silicate or talc. According to a number of embodiments, the disclosed composition comprises memantine and melatonin, one or more diluents, each being independently selected from starch, lactose monohydrate or microcrystalline cellulose, one or more disintegrating agents, each being independently selected from jellied starch or carboxymethyl cellulose, a binding agent and an antifriction agent being a lubricant. According to other preferred embodiments, the binding agent is polyvinylpyrrolidon, and the lubricant is magnesium stearate. According to still other embodiments, but not exclusive ones, the diluent may be lactose (in the monohydrate form), the disintegrating agent may be cross-carmellose (in the sodium salt form), and the binding agent is povidone.

Pharmaceutically acceptable adjuvants are selected for the purpose of ensuring delivery of the therapeutically efficient, in a single dose, amount of memantine and melatonin in a common single dosage form and for the purposes of optimizing costs, simplifying and stabilizing the production process. The necessary conditions for the adjuvants are their inertness and chemical and physical compatibility with memantine and melatonin. Adjuvants used in solid dosage forms, such as tablets and capsules, may also include colorants and pigments, substances masking taste, flavoring agents, sweeteners and adsorbents. Diluents promote an increase in the tablet size with a small amount of an active pharmaceutical substance. Diluents include lactose in the forms of alpha-lactose or beta-lactose. Different types of lactose may consist of lactose monohydrate, alpha-lactose monohydrate, anhydrous alpha-lactose, anhydrous beta-lactose and agglomerated lactose. Other diluents may include sugars, such as sucrose, inverted sugar, dextrose and dextrats. Lactose monohydrate is the more preferred diluent. Another diluent may be microcrystalline cellulose, including micronized one.

Diluents may include starch and starch derivatives. Starches include natural starches produced from various cereal crops and/or other agricultural crops. Also, starches may include pre-jellied starch and starch modified with sodium glycolate. Starches and starch derivatives also possess the property of disintegrating agents. Many diluents also act as disintegrating agents and binding agents, and these additional properties should be taken into account when producing a pharmaceutical composition. Disintegrating agents are added for the purpose of destructing a tablet into particles of an active pharmaceutical component and adjuvants, in order to promote dissolution and improve biological availability of therapeutically active ingredients. Starch and starch derivatives, including a sodium salt of starch carboxymethyl ester, such as, for example, starch modified with sodium glycolate, are applicable disintegrating agents.

Jellied starch may be a preferred, but not exclusive, disintegrating agent. Another preferred disintegrating agent is sodium carboxymethyl cellulose.

Binding agents are used as pharmaceutically acceptable adjuvants for wet granulation, in order to raise a concentration of therapeutically active substances and other adjuvants in granules. A binding agent is added in order to improve fluidity of a powder and quality of pressing. Binding agents include cellulose derivatives, such as microcrystalline cellulose, methyl cellulose, carboxymethyl cellulose, hydroxypropylmethyl cellulose, hydroxyethyl cellulose and hydroxypropyl cellulose. Other binding agents are selected from such substances as povidone, polyvinylpyrrolidon, gelatin, a natural gum, namely, acacia, tragacanth, guar and pectin gums, starch paste, pre-jellied starch, polyethylene glycols and sodium alginate.

Polyvinylpyrrolidon, in particular povidone is a preferred binding agent.

Antifriction agents are lubricating and sliding substances that are used in the production of solid dosage forms for the purpose of preventing a tablet from adhering to process surfaces and reducing adhering during pressing stages. Such substances include stearic acid, stearic acid salts, for example, calcium stearate, magnesium stearate and sodium stearyl fumarate, talc, sodium benzoate, sodium acetate and sodium oleate. The choice for a lubricating agent, but not exclusive, is magnesium stearate.

In order to improve fluidity, lubricating substances are used in solid dosage forms for the purpose of reducing friction between particles. Lubricating substances have properties of both diluents and solidifying components. According to still further embodiments of the composition of memantine and melatonin, an active substance content is approximately 30-50% based on the combination weight. Preferably, his composition comprises a diluent that is lactose monohydrate, but not exclusively, a second diluent that is microcrystalline cellulose, but not exclusively, a disintegrating agent that is jellied starch, but not exclusively, a second disintegrating agent that is carboxymethyl cellulose, but not exclusively, a binding agent that is polyvinylpyrrolidon, but not exclusively, and a lubricating agent that is magnesium stearate, but not exclusively. According to still further preferred embodiments, lactose monohydrate forms approximately 25-40% based on the composition weight, microcrystalline cellulose forms approximately 5-15%, jellied starch forms approximately 5-10%, sodium carboxymethyl cellulose forms approximately OT 1-5%, polyvinylpyrrolidon forms approximately 1-5% and magnesium stearate forms approximately 0.2-2.0%.

According to several most preferable embodiments, the active source forms approximately 40.0% based on the composition weight, lactose monohydrate forms approximately 28.7%, microcrystalline cellulose forms approximately 10.4%, starch forms approximately 10.9%, carboxymethyl cellulose forms approximately 4.0%, polyvinylpyrrolidon forms approximately 5.2% and magnesium stearate forms approximately 0.8%.

According to still further embodiments, memantine and melatonin form approximately 70-80% based on the composition weight. Preferably, this composition comprises a diluent, such as lactose monohydrate, preferably approximately 3-20% based on the composition weight; a disintegrating agent, such as, for example, cross-linked sodium carboxymethyl cellulose, preferably approximately 2-10% based on the composition weight; a binding agent, such as, for example, polyvinylpyrrolidon, preferably approximately 2-10% based on the composition weight; and lubricating agent, such as, for example, magnesium stearate, preferably approximately 0.2-2.0% based on the composition weight.

According to still further embodiments, memantine and melatonin form approximately 80% based on the composition weight, the diluent is lactose monohydrate that forms 8-15% based on the composition weight; the disintegrating agent is carboxymethyl cellulose that forms 1-10% based on the composition weight; the binding agent is polyvinylpyrrolidon that forms 1-10% based on the composition weight; and the antifriction lubricating agent is magnesium stearate that forms 0.2-2.0% based on the composition weight.

According to several more preferred embodiments, the diluent is lactose monohydrate and it may form approximately 9.5% based on the composition weight, the disintegrating agent is cross-carmellose and it may form approximately 5% based on the composition weight, the binding agent is povidone and it may form approximately 5% based on the composition weight, and the lubricating agent is magnesium stearate and it may form approximately 0.5% based on the composition weight.

According to other embodiments, memantine and melatonin form approximately 90% based on the composition weight. Preferably, but not exclusively, this composition comprises the diluent, such as lactose monohydrate, ranging within 3-10% based on the composition weight; the disintegrating agent, for example, carboxymethyl cellulose, preferably approximately 2-5% based on the composition weight; the binding agent, such as, for example, polyvinylpyrrolidon, preferably approximately 2-5% based on the composition weight; and the lubricating agent, for example, magnesium stearate, preferably approximately 0.2-2.0% based on the composition weight. According to other more preferable embodiments the diluent is lactose monohydrate, and it may form approximately 3.5% based on the composition weight, the disintegrating agent is cross-carmellose and it may form approximately 3% based on the composition weight, the binding agent is povidone and it may form approximately 3% based on the composition weight, and the lubricating agent is magnesium stearate and it may form approximately 1% based on the composition weight.

According to another embodiment, the proposed invention provides for the memantine content of 50 mg, 100 mg or 150 mg and the melatonin content of 3 mg, 5 mg or 10 mg, wherein memantine/melatonin form from 45 to 90% based on the composition weight. According to still further embodiments, memantine/melatonin form approximately from 60 to 90% or 70-80% based on the composition weight. According to another embodiment, this composition comprises one or more starches, such as corn starch, lactose monohydrate, microcrystalline cellulose, jellied starch, carboxymethyl cellulose; sodium salt of starch carboxymethyl ester; polyvinylpyrrolidon, hydroxymethylpropyl cellulose; magnesium stearate; and a mineral salt, such as talc. According to still another embodiment, this composition comprises lactose monohydrate, corn starch, sodium carboxymethyl cellulose, polyvinylpyrrolidon, talc and magnesium stearate.

According to yet another embodiment this composition comprises lactose monohydrate, microcrystalline cellulose, jellied starch, carboxymethyl cellulose, polyvinylpyrrolidon and magnesium stearate. According to still another embodiment, this composition comprises lactose monohydrate, sodium carboxymethyl cellulose, polyvinylpyrrolidon and magnesium stearate.

Preferably, standard doses of memantine and melatonin are in the form of a solid dosage form, such as a tablet or capsule, and more preferably a tablet. In particular, this tablet may comprise 30 mg, 50 mg and/or, preferably, 100 mg of memantine and 3 mg, 5 mg, 10 mg of melatonin in a tablet having the weight of 250 mg. According to other embodiments, the tablet may comprise up to 150 mg of memantine and up to 5 mg of melatonin in a 500mg tablet, 200 mg of memantine and up to 10 mg of melatonin in a 700mg tablet and 200 mg of memantine and up to 20 mg of melatonin in a 1,000mg tablet.

According to yet further embodiments, the tablet may comprise 100 mg of memantine and up to 5 mg of melatonin in a 150mg tablet, 200 mg of memantine and up to 10 mg of melatonin in a 250mg tablet, 300 mg of memantine and up to 5 mg of melatonin in a 375mg tablet, and 400 mg in a 500mg tablet.

According to still further embodiments of this dosage form, it may comprise 100 mg of memantine and up to 3 mg of melatonin in a 125mg (+/- 3 mg) tablet, 150 mg of memantine and up to 5 mg of melatonin in a 225mg (+/-5 mg) tablet, 200 mg of memantine and up to 10 mg of melatonin in a 340mg (+/- 5 mg) tablet, and 250 mg of memantine and up to 15 mg of melatonin in a 450mg tablet of memantine and up to 10 mg of melatonin. A capsule may comprise 25 mg, 50 mg or 100 mg of memantine and up to 3 mg of melatonin in a 125mg capsule, or 200 mg of memantine and up to 10 mg of melatonin in a 250mg capsule. In a similar way, a capsule may also comprise 100 mg of memantine and up to 3 mg of melatonin in a 115mg capsule, or 200 mg of memantine and up to 10 mg of melatonin in a 230mg capsule.

According to one embodiment, this invention provides a method for producing a solid dosage form of memantine and melatonin with the use of wet mixing of memantine and melatonin and adjuvants with water, drying and grinding of a granulated mixture. According to this embodiment, the finished mixture is pressed for producing tablets. According to other embodiments, the finished mixture may be used for capsulation.

The process of producing a tablet according to this invention is supposed to be as follows.

Memantine, melatonin and lactose are loaded into a reactor. The produced mixture is fed into a mixer wherein carboxymethyl cellulose and starch are successively added in a ratio suitable for producing a gelatinized starch solution and a powder for dusting. This is moistened with a water solution of polyvinylpyrrolidon. A composition of the active substances and adjuvants is optimal for implementing this embodiment of the invention and enables to produce quality tablets corresponding to the Pharmacopeia requirements. After mixing, the mass in the mixer is moistened with the gelatinized starch solution.

The strength parameters and tablet decomposition are controlled by changing the concentration of gelatinized starch. Then, the tablet-mass is unloaded from the mixer and dried in a boiling bed. A finished mass is granulated by feeding it onto a sieve having holes of 1.2-2 mm and loaded into the mixer, then a mixture of starch and calcium stearate are added, and all the components are mixed. The granulated matter is fed for producing tablet mass. A dry granulated matter is loaded into the mixer, calcium stearate is added, and all the components are mixed for 1-2 minutes. The dusted tablet-mass is unloaded from the reactor and fed to the stage of producing tablets. Tablets are produced on a rotary press with the use of round dies.

### Best Mode of Carrying out the Invention

### Example 1. Composition of memantine/melatonin tablets (Table 1).

**Table 1.**

| **Substance** | **mg** | **Weight %** |
|---|---|---|
| Memantine | 100 | 40.00 |
| Melatonin | 5 | 2.00 |
| Lactose | 70 | 28.00 |
| Microcrystalline cellulose | 24.75 | 9.90 |
| Starch | 26 | 10.40 |
| Povidone | 12.5 | 5.00 |
| Cross-carmellose | 9.75 | 3.90 |
| Calcium stearate | 2 | 0.80 |
| **Tablet total weight** | **250** | **100.00** |

### Example 2. Composition of memantine/melatonin tablets (Table 2).

**Table 2.**

| **Substance** | **mg** | **Weight** % |
|---|---|---|
| Memantine | 100 | 83.332 |
| Melatonin | 5 | 4.167 |
| Lactose | 5.25 | 4.375 |
| Microcrystalline cellulose | 1.25 | 1.042 |
| Starch | 2.25 | 1.875 |
| Povidone | 2 | 1.667 |
| Cross-carmellose | 2.25 | 1.875 |
| Calcium stearate | 2 | 1.667 |
| **Tablet total weight** | **120** | **100.00** |

### Example 3. Compositions with high dose of memantine and melatonin (Table 3).

**Table 3.**

| **Substance** | **mg** | **Weight %** | **mg** | **Weight %** |
|---|---|---|---|---|
| Memantine | 100.00 | 89.83 | 200.00 | 88.95 |
| Melatonin | 3.00 | 2.69 | 10.00 | 4.45 |
| Lactose | 3.00 | 2.69 | 4.50 | 2.00 |
| Povidone | 2.00 | 1.80 | 4.12 | 1.83 |
| Cross-carmellose | 2.12 | 1.91 | 4.12 | 1.83 |
| Calcium stearate | 1.20 | 1.08 | 2.10 | 0.94 |
| **Tablet total weight** | **111.32** | **100** | **224.84** | **100** |

The proposed invention may be implemented by producing the final medication as capsules, preferably solid gelatin capsules. Solid gelatin capsules may be produced by filling the long portion of a capsule with a prepared mixture and capsuling the long portion of a capsule by a machine mode. According to this way of implementing the proposed invention, a method of producing the finished dosage form of memantine with melatonin is realized. For this, memantine and melatonin are dry-mixed with adjuvants. According to one embodiment, sifted powders of lactose, carboxymethyl cellulose, memantine and melatonin, starch are loaded into a mixer and mixed thoroughly. Sifted powder of colloid silicon dioxide is added to the produced mass, mixed, then sifted magnesium stearate is added. The produced mixture is mixed thoroughly. The produced mass is supplied for capsuling.

### Example 4. Composition of memantine/melatonin capsules (Table 4).

**Table 4.**

| **Substance** | **mg** | **Weight %** | **mg** | **Weight %** |
|---|---|---|---|---|
| Memantine | 50 | 40.13 | 100 | 80.0 |
| Melatonin | 3 | 2.41 | 5 | 4.0 |
| Lactose | 59.5 | 47.75 | 9.25 | 7.4 |
| Povidone | 5.75 | 4.62 | 2 | 1.6 |
| Cross-carmellose | 5.75 | 4.61 | 6.75 | 5.4 |
| Calcium stearate | 0.6 | 0.48 | 2 | 1.6 |
| **Capsule total weight** | **124.6** | **100** | **125** | **100** |

It should be mentioned that solid dosage forms may include, according to the proposed invention, tablets and capsules as well as drops, pastilles, powders, starch capsules, etc.

Other aspects of the proposed invention also relate to the use of these compositions for prophylaxis and treatment of diseases or disorders in a mammal in need thereof, wherein such use includes administration of a therapeutically efficient amount of a composition according to this invention to such a mammal. In particular, these compositions are suitable for prophylaxis and treatment of mental, behavioral, and cognitive disorders.

### Example 5. Study of pharmacological activity.

The subjects under the study have been 25 patients with mixed dementia and 12 patients with Alzheimer's disease. The average age of the patients is 70±3 years. Dementia has been diagnosed in accordance with the dementia criteria under ICD-10 [International statistic classification of diseases and problems relating to health. Tenth revision (ICD-10). V.1 (Part 1). Geneva: WHO, 1995; p.315, 510-1]. Dimentia severity has been assessed according to Clinical Dementia Rating Scale - CDR [Hughes CP, Berg L, Danziger WL et al. A new clinical scale for the staging of dementia. Brit J Psychiatry 1982; 140: 566-72]. In the group of patients with mixed dementia it is 1.2±0.3, and in the group with Alzheimer's disease - 1.1±0.36. All the patients have been subdivided into two groups. The first group has received 50 mg of memantine once a day. The second group received capsules according to Example 4. A complex neurological study has been conducted with the patients for half a year, the study included brief evaluation of mental status (MMSE), a test for visual and aural-speech memory under the dementia scale, the clock drawing test as well as the test for attention - Schulte Table. The outcome has been processed with the use of the statistical package for social sciences (SPSS), Version 10.0.

Results: in 1.5 months after the beginning of administering the preparation the patients showed reliable reduction in manifestation of cognitive disorders. In 6 months the total score of the mental status evaluation in the first group increased by 1.5±0.3, visual memory by 0.5±0.2, the clock drawing test by 0.2±0.1, the Schulte test lowered by 20.5±3.6; in the second group, respectively: by 2.4±0.5, 0.8±0.2, 0.4±0.1, 36.2±4.5.

For two patients from the first group it became necessary to lower the dose, since an increase in the intracranial pressure was observed.

A positive therapeutic effect was recorded for 25% of the patients with Alzheimer's disease and 86% of the patients with mixed dementia from the first group. A positive effect in the second group was observed for 40% of the patients with Alzheimer's disease 40% and 92% of the patients with mixed dementia, no pronounced side effects being observed. The state stabilization effect was observed in the other patients.

Thus, a significant increase in the memantine effect is clearly seen, when it is combined with melatonin.

The materials, methods and examples, as presented herein, are purely illustrative and in no way should be interpreted as limiting the scope and/or essence of the proposed invention, except for a case where technological, scientific and experimental data is well-known in the field of use of the disclosed invention.

### Industrial Applicability

The invention relates to the field of medicine and chemical-pharmaceutical industry, namely, to the field of neurology, and concerns new compositions comprising memantine and melatonin that are used for prophylaxis and treatment of mental, behavioral, and cognitive disorders.

## Claims

1. A pharmaceutical composition for use in the prophylaxis and treatment of mental, behavioral, and cognitive disorders in the form of a solid dosage form, **characterized in that** it comprises memantine and melatonin as the active source and adjuvants including at least one diluent selected from lactose, starch, starch derivative, microcrystalline cellulose, sucrose, inverted sugar, decstrose and decstrate; at least one disintegrating agent selected from sodium carboxymethyl cellulose, cross-carmellose, jellied starch; a binding agent selected from polyvinylpyrrolidon, gelatin, cellulose derivatives, natural gums, polyethylene glycols, sodium alginate; an antifriction agent selected from stearic acid and/or its salts, colloid silicon dioxide, talc, sodium benzoate, sodium acetate, and sodium oleate, wherein the content of the components, in weight %, is as follows:
memantine 40.0 - 90.0
melatonin 2.0 - 5.0
diluent 2.0 - 50.0
binding agent 3.5 - 10.0
disintegrating agent 1.5 - 10.0
antifriction agent 0.2 - 3.0.

2. The pharmaceutical composition for use according to Claim 1, **characterized in that** it comprises a combination of memantine and melatonin in an amount of 70-80% based on the composition weight.

3. The pharmaceutical composition for use according to Claim 1, **characterized in that** it comprises a combination of memantine and melatonin in an amount of 70-80% based on the composition weight, the diluent - lactose monohydrate - in an amount of 3-20% based on the composition weight; the disintegrating agent - cross-linked sodium carboxymethyl cellulose in an amount of 2-10% based on the composition weight; the binding agent - polyvinylpyrrolidon - in an amount of 2-10% based on the composition weight; and the antifriction agent - magnesium stearate - in an amount of 0.2-2.0% based on the composition weight.

4. The pharmaceutical composition for use according to Claim 1, **characterized in that** comprises a combination of memantine and melatonin in an amount of 60-90% based on the composition weight.

5. The pharmaceutical composition for use according to Claim 1, **characterized in that** it is in the form of a tablet.

6. The pharmaceutical composition for use according to Claim 5, **characterized in that** it comprises approximately 100 mg of memantine and 5 mg of melatonin.

7. The pharmaceutical composition for use according to Claim 5, comprising approximately 150 mg of memantine and 10 mg of melatonin.

8. The pharmaceutical composition for use according to Claim 5, comprising approximately 30 mg of memantine and 3 mg of melatonin.

9. The pharmaceutical composition for use according to Claim 1, **characterized in that** it is in the form of a solid capsule.

10. The pharmaceutical composition for use according to Claim 1, **characterized in that** it comprises, in weight %:
memantine 89.83%
melatonin 2.69%
lactose 2.69%
povidone 1.80%
cross-carmellose 1.91%
calcium stearate 1.08%
